# EUROPÄISCHE PATENTSCHRIFT

(11) **EP 3 614 937 B1**
(45) Veröffentlichungstag und Bekanntmachung des Hinweises auf die Patenterteilung: **12.04.2023**
(21) Anmeldenummer: 18722009.0
(22) Anmeldetag: 27.04.2018
(51) Int. Cl.: A61B 17/3201, A61B 17/28, A61B 90/00

(54) **CHIRURGISCHES INSTRUMENT DER ZWEI BRANCHEN-BAUART MIT VERBESSERTER REINIGUNGSEIGNUNG**
SURGICAL INSTRUMENT OF TWO-BRANCH DESIGN WITH IMPROVED CLEANING SUITABILITY
INSTRUMENT CHIRURGICAL DE CONSTRUCTION À DEUX BRANCHES AVEC FACILITÉ DE NETTOYAGE AMÉLIORÉE

(30) Priorität: 27.04.2017 DE 102017109125
(43) Veröffentlichungstag der Anmeldung: 04.03.2020
(73) Patentinhaber: Aesculap AG, 78532 Tuttlingen (DE)
(72) Erfinder: ETTWEIN, Pierre, 78234 Engen (DE); VOGTHERR, Robert, 78532 Tuttlingen (DE)
(74) Vertreter: Winter, Brandl - Partnerschaft mbB
(86) Internationale Anmeldenummer: PCT/EP2018/060894
(87) Internationale Veröffentlichungsnummer: WO 2018/197679

(56) Entgegenhaltungen:
- EP-A1- 2 594 210
- CN-U- 202 723 937
- CN-U- 203 436 369
- JP-A- 2005 204 997

## Beschreibung

Die Erfindung betrifft ein scheren- oder zangenartiges medizintechnisches Instrument mit einer ersten Instrumentenbranche (Instrumententeil), bestehend aus einem distalen Eingriffs-/Wirkabschnitt, einem proximalen Griff-/Halteabschnitt und einem dazwischen angeordneten Kopplungs-/Schlussabschnitt, einer zweiten Instrumentenbranche (Instrumententeil) ebenfalls bestehend aus einem distalen Eingriffs-/Wirkabschnitt, einem proximalen Griff-/Halteabschnitt und einem dazwischen angeordneten Kopplungs-/Schlussabschnitt und einem die Instrumentenbranchen (Instrumententeile) in einem aufgelegten Schluss koppelnden Lagerelement, wodurch die beiden Instrumentenbranchen (Instrumententeile) relativ zueinander scherenartig schwenkbar sind und sich in einem Arbeitsschwenkbereich über einander zugewandte Kontaktflächen in den jeweiligen Kopplungs-/Schlussabschnitten gegenseitig (gleitend) kontaktieren, so dass die beiden Instrumentenbranchen in dem Arbeitsschwenkbereich aneinander mit ihren jeweiligen Kontaktflächen lagernd und führend (d.h. gleitend) anliegen, und die Instrumentenbranchen in eine Reinigungsstellung außerhalb des Arbeitsschwenkbereichs positionierbar sind, wobei das Lagerelement vorzugsweise gewindelos ist und an einem Endabschnitt unmittelbar an der ersten Instrumentenbranche fest fixiert ist und am anderen Endabschnitt einen sich mit zunehmendem Abstand von der ersten Instrumentenbranche kontinuierlich konisch oder gestuft konisch aufweitenden Kopf aufweist, der eine kontinuierlich oder gestuft konische Durchgangsöffnung in der zweiten Instrumentenbranche durchgreift, wobei die Durchgangsöffnung in Form eines Langlochs ausgebildet ist.

### Stand der Technik

Bei bekannten zweiteiligen medizintechnischen / chirurgischem Standard-Instrumenten der Scheren- und/oder Zangenbauart kommen einerseits sogenannte aufgelegte Schlüsse und anderseits sogenannte Durchsteck-Schlüsse als Gelenk zwischen zwei Instrumentenbranchen (Instrumententeilen) zum Einsatz. Beide Versionen besitzen unter anderem den Nachteil, dass sich zwischen den schwenkbar miteinander gekoppelten Instrumentenbranchen verdeckte Spaltflächen im Gelenkbereich befinden, die infolge der Kopplung der Instrumente in keiner (Winkel-)Stellung der Branchen zueinander freiliegen oder zugänglich sind. Derartig verdeckte Spaltflächen ermöglichen keine optimale Reinigung und Sterilisation der Instrumente. Im Allgemeinen werden die Branchen der Instrumente zur Reinigung in eine um etwa 90° geöffnete Stellung gebracht, so dass die verdeckten Flächen etwas reduziert sind. Ein optimales Reinigungsergebnis ist jedoch auch dann nur schwer möglich.

Es hat unterschiedliche Ansätze gegeben, das Problem von schlecht oder nicht zugänglichen Spaltflächen zwischen den Branchen gattungsgemäßer medizintechnischer Instrument zu verringern oder zu beseitigen. So ist zum Beispiel aus der EP 2 594 210 A1 ein scheren- oder zangenartiges medizinisches Instrument mit einer ersten und einer zweiten Branche (Instrumententeil) bekannt, die über einen mit einem radial erweiterten Kopfteil einerseits und einem Schraubengewinde andererseits versehenen Lagerzapfen schwenkbar miteinander in Verbindung stehen. Die erste Branche bildet eine Führungsplatte und die zweite Branche eine Lagerplatte aus, in die der Lagerzapfen eingeschraubt ist. Die Führungsplatte weist zur schwenkbaren Aufnahme des Lagerzapfens einen Durchbruch auf. Die Führungsplatte und die Lagerplatte berühren einander im normalen Betriebszustand des Instrumentes flächig. Der Durchbruch der Führungsplatte ist als sich in Längsrichtung der Führungsplatte erstreckendes Langloch (d.h. durchgreifende Längsnut) ausgebildet, das im Randbereich seines dem Werkzeugteil benachbarten Endbereichs eine erste erweiterte Vertiefung und im Randbereich seines in Längsrichtung gegenüber liegenden Endbereichs eine zweite erweiterte Vertiefung aufweist, in welchen das Kopfteil des Lagerzapfens wahlweise aufnehmbar ist. Die erste Vertiefung besitzt eine geringere Tiefe (T1) als die zweite Vertiefung.

Die Lagerplatte weist auf ihrer, der Führungsplatte zugewandten, Kontaktfläche eine im Bereich des Lagerzapfens angeordnete, quer zur Lagerplatte verlaufende Quernut (Einbuchtung) auf, deren Tiefe (T3) zumindest der Tiefe (T1) der ersten Vertiefung des Langloches entspricht und deren Breite (b) zumindest der Breite (B) der Führungsplatte entspricht. Die erste Branche ist mit ihrer Führungsplatte in eine mit der Quernut fluchtende Schwenkposition bringbar und in Richtung hin zum Lagerzapfen in die Quernut soweit verstellbar, dass das Kopfteil des Lagerzapfens mit der ersten Vertiefung des Langloches außer Eingriff gelangt. Das Kopfteil des Lagerzapfens ist durch eine Verstellung der zweiten Branche mit seiner Lagerplatte und dem Lagerzapfen entlang des Langloches mit der zweiten Vertiefung des Langloches in Überdeckung und durch eine Verstellung in Richtung des Lagerzapfens mit der zweiten Vertiefung in Eingriff zu bringen.

Die Führungsplatte und die Lagerplatte weisen in dieser Position einen Abstand voneinander auf. Bei diesem Instrument ist von Nachteil, dass die beiden Branchen zur Reinigung relativ kompliziert zueinander verschoben werden müssen, wobei zunächst der Lagerzapfen und die diesen aufnehmende erste Vertiefung außer Eingriff gebracht werden müssen. Dies ist unter Umständen mit unerwünschtem Verschleiß der Lagerung verbunden. Außerdem ist aufgrund der reduzierten Senktiefe für den Schraubenkopf des Lagerzapfens die Drehführung / die Stabilität des Schlusses begrenzt, was nur durch eine höhere Schlussdicke mit entsprechenden Nachteilen bei der Anwendung insbesondere Schwergängigkeit bei der Relativverschwenkung der beiden Branchen auszugleichen ist. Diese Lösung scheint daher für Scheren akzeptabel, für höher belastete Instrumente wie Zangen oder dergleichen jedoch weniger gut geeignet zu sein.

Die EP 2 873 381 A1 offenbart ein medizinisches Instrument bestehend aus einem ersten eine Führungsplatte aufweisenden Handhebel (Instrumententeil) und einem zweiten eine Lagerplatte aufweisenden Handhebel (Instrumententeil), die über einen im Bereich der Führungsplatte und der Lagerplatte angeordneten und mit einem radial erweiterten Kopfteil versehenen Lagerzapfen schwenkbar miteinander verbunden/gekoppelt sind. Die Lagerplatte bildet in ihren längseitigen Randbereichen jeweils eine Aufnahmenut, in welche die Führungsplatte in ihrer Betriebsstellung eingreift. Die Lagerplatte weist zwischen den Aufnahmenuten eine sich über die komplette Breite der Lagerplatte erstreckende Vertiefung auf. Die Führungsplatte ist aus ihrer Betriebsstellung in eine Reinigungsstellung relativ zur Lagerplatte drehbar, in welcher die Führungsplatte mit der Vertiefung an der Lagerplatte in Überdeckung gelangt. Im Bereich der Vertiefung ist zwischen der Führungsplatte und der Lagerplatte ein Distanzelement vorgesehen, dessen Höhe in Richtung des Lagerzapfens gleich groß oder kleiner ist als die Tiefe der Vertiefung, sodass die Führungsplatte in der die Vertiefung überdeckenden Winkelposition nicht auf den Vertiefungsgrund absinken kann.

Bei diesem Instrument ist von Nachteil, dass eine Sichtkontrolle des Reinigungserfolgs im Schlussspalt in der Reinigungsstellung nur begrenzt möglich ist. Außerdem bilden die Randbereiche der Lagerplatte Übergänge oder Stufen aus, welche beim Präparieren stören können (z.B. Knoten von Nahtmaterial, Einklemmen von Gewebe).

Aus der EP 2 412 324 A2 ist ein ähnliches Instrument bekannt, bei dem ein Gelenkzapfen eine zweite Lagerplatte durchragt und in seinem die zweite Lagerplatte durchragenden Endbereich mit einem radial erweiterten Ringflansch versehen ist. Dieser weist von einer ersten Lagerplatte einen Abstand (a) auf, der der doppelten Dicke (d) der auf dem Gelenkzapfen gelagerten zweiten Lagerplatte entspricht oder größer ist als die doppelte Dicke (d) der zweiten Lagerplatte so dass der zweite Handhebel (Instrumententeil) in einer geöffneten Schwenklage zum ersten Handhebel (Instrumententeil) entlang des Gelenkzapfens in eine Lage verschiebbar ist, in der die einander gegenüber liegenden Kontaktflächen der abgeflachten Lagerplatten frei liegen. Bei diesem Instrument ist jedoch von Nachteil, dass der Gelenkzapfen über den Schlussabschnitt vorsteht. Auch hier bilden die Randbereiche der Lagerplatte Übergänge oder Stufen aus, welche beim Präparieren stören können (z.B. Knoten von Nahtmaterial, Einklemmen von Gewebe).

Die DE 297 12 016 U1 offenbart eine zerlegbare Mehrzweckschere, bei die Instrumententeile lösbar gekoppelt sind. Aus der DE 20 2004 002 560 U1 ist ebenfalls eine zerlegbare Dental-Zange bekannt. Bei diesen Instrumenten bewirkt die Zerlegbarkeit des Instruments einen Mehraufwand. Außerdem ist bei der Wiedermontage nach einer Reinigung eine korrekte Paarung von Instrumentenhälften sicherzustellen. Es besteht in nachteiliger Weise auch eine gewisse Verwechslungsgefahr bei unterschiedlichen Instrumentengrößen.

Aus der DE 201 00 589 U1 ist schließlich ein chirurgisches Instrument mit zwei schwenkbar miteinander verbundenen Branchen bekannt, die im Bereich ihrer Schwenkverbindung ebene, beim Verschwenken der Branchen in ihrem Arbeitsbereich aneinander anliegende Anlageflächen aufweisen. Es sind Mittel vorgesehen, die bei einer relativen Verschiebung der beiden Branchen aus ihrem Arbeitsbereich heraus die flächige Anlage der Anlageflächen bereichsweise aufheben. Bei der dort offenbarten Ausführungsform mit in eine Reinigungsstellung verschiebbaren Branchen ist die Verschleißanfälligkeit nachteilig, wobei auch keine/schlechte Einsehbarkeit im Schlussabschnitt zur Sichtkontrolle des Reinigungserfolgs besteht. Bei der Ausführungsform mit entfernbarem Lagerstift ist der Montageaufwand ferner hoch und der Lagerstift kann zudem verloren werden. Bei einem aufgelegten Schraubschluss mit Reinigungsspalt ist eine Sichtkontrolle des Reinigungserfolgs in Schlussspalt in der Reinigungsstellung nur begrenzt möglich. Die Schraubverbindung ist als solche im Hinblick auf eine Reinigungseignung und/oder die Korrosionsbeständigkeit (u.a. Spannungsrisskorrosion) ggf. nachteilig.

CN 202723937 U offenbart eine Schere mit einer ersten Branche und einer zweiten Branche, die mittels einer Buchse und einem Lagerzapfen miteinander schwenkbar gekoppelt werden können.

Es sind im Übrigen Instrumente bekannt, bei denen eine Schlussschraube auf Anschlag (Bund) in eine der Instrumentenbranchen eingeschraubt ist und zur Sicherung leicht überdreht ist, wodurch es zu einem Kaltverschweißen der Gewindegänge kommt. Die Schlussschraube durchgreift eine Stufenbohrung in der anderen Instrumentenbranche und weist einen Schraubenkopf auf, der an der anderen Instrumentenbranche zur Anlage kommt und diese in Richtung der einen Instrumentenbranche drängt / verspannt. Der Gang des Instruments ergibt sich (resultiert aus Verspannung der Instrumentenbranchen gegeneinander) aus dem Abstand der Anlagefläche des Schraubenkopfs zur Kontaktfläche der einen Instrumentenbranche sowie den Toleranzen der Stufenbohrung der anderen Instrumentenbranche. Dadurch ist die Verschraubung überbestimmt. In der Praxis müssen deshalb oft Schlussschrauben minimal unterschiedlicher Längen bereitgehalten werden, um bei Bedarf Fertigungstoleranzen der Einzelteile auszugleichen. Dies ist mit erhöhtem Aufwand / erhöhten Kosten verbunden. Zudem Bedarf das manuelle Setzen der Schlussschrauben einiges an Erfahrung, um das optimale Anzugsmoment zu treffen. Ein automatisiertes, drehmomentgesteuertes Anziehen der Schlussschrauben ist nur bedingt möglich, aufgrund der unterschiedlichen Einflussfaktoren. Es sind zudem Instrumente bekannt, bei denen die Schlussschraube per Schweißpunkt oder durch Verkleben gesichert wird, um ein Lösen zu verhindern. Nachteilig kann hierbei eine aus dem Schweißpunkt resultierende Korrosionsunbeständigkeit sein.

### Kurzbeschreibung der Erfindung

Die der Erfindung zugrundeliegende Aufgabe ist demnach ein chirurgisches Instrument zu schaffen, das die vorstehend beschriebenen Nachteile zumindest teilweise beseitigt, insbesondere einen Instrumentenschluss mit guter Reinigungseignung zu entwickeln, welche auch intuitiv erkennbar ist. Neben der leichten Reinigbarkeit soll bevorzugt auch eine gute Sichtkontrolle des Reinigungserfolgs möglich sein. Der Fertigungsaufwand und die Herstellungskosten sollen vorzugsweise möglichst gering sein. Schließlich sollte die Funktionsfähigkeit des Instruments, insbesondere die leichtgängige, jedoch präzise Führung der beiden aneinander scharnierten Branchen (Instrumententeile) gewährleistet sein.

Diese Aufgabe sowie die bevorzugt gestellten Ziele werden nach der Erfindung gelöst bzw. erreicht mittels eines (scheren- oder zangenartigen) Instruments gemäß Anspruch 1, insbesondere durch ein scheren- oder zangenartiges medizintechnisches Instrument mit einer ersten Instrumentenbranche (Instrumententeil), bestehend aus einem distalen Eingriffs-/Wirkabschnitt, einem proximalen Griff-/Halteabschnitt und einem dazwischen angeordneten Kopplungs-/Schlussabschnitt, einer zweiten Instrumentenbranche (Instrumententeil) ebenfalls bestehend aus einem distalen Eingriffs-/Wirkabschnitt, einem proximalen Griff-/Halteabschnitt und einem dazwischen angeordneten Kopplungs-/Schlussabschnitt und einem die Instrumentenbranchen (Instrumententeile) in einem aufgelegten Schluss koppelnden Lagerelement, wodurch die beiden Instrumentenbranchen (Instrumententeile) relativ zueinander scherenartig schwenkbar sind und sich in einem Arbeitsschwenkbereich über einander zugewandte Kontaktflächen in den jeweiligen Kopplungs-/Schlussabschnitten gegenseitig (gleitend) kontaktieren, so dass die beiden Instrumentenbranchen in dem Arbeitsschwenkbereich aneinander mit ihren jeweiligen Kontaktflächen lagernd und führend (d.h. gleitend) anliegen, und die Instrumentenbranchen in eine Reinigungsstellung außerhalb des Arbeitsschwenkbereichs positionierbar sind, wobei das Lagerelement vorzugsweise gewindelos ist und an einem Endabschnitt unmittelbar an der ersten Instrumentenbranche fest fixiert ist und am anderen Endabschnitt einen sich mit zunehmendem Abstand von der ersten Instrumentenbranche kontinuierlich konisch oder gestuft konisch aufweitenden Kopf aufweist, der eine kontinuierlich oder gestuft konische Durchgangsöffnung in der zweiten Instrumentenbranche durchgreift, wobei die Durchgangsöffnung in Form eines Langlochs ausgebildet ist, wobei das Lagerelement in dem Langloch drehpositionierbar und translatorisch fest aufgenommen ist, insbesondere in Längsrichtung des Langlochs positionsfest aufgenommen ist.

Vorzugsweise ist der Lagerzapfen gewindelos und wird an der einen Branche stoff- oder reibschlüssig fixiert, beispielsweise vernietet, verschweißt, verpresst und/oder verklebt. Er kann aber auch ein Gewinde aufweisen. In diesem Fall kann die Schraubverbindung vorzugsweise drehmomentgesteuert angezogen sein. Dadurch ergibt sich eine definierte Vorspannkraft zwischen den Branchen. Die Schraubverbindung kann zudem verklebt, verschweißt oder verstemmt werden.

Unter dem Arbeitsschwenkbereich ist im Sinne der Erfindung ein solcher Schwenkbereich (Schwenkwinkelbereich) zu verstehen, in dem die beiden Instrumentenbranchen bei einer bestimmungsgemäßen Verwendung zueinander relativ verschwenkt werden. Dabei ist sichergestellt, dass die beiden Branchen im gesamten vorbestimmten Arbeitsschwenkbereich an ihren Kontaktflächen aneinander (spielfrei) anliegen, sowie dass der konische/(mehrfach) gestufte Kopf des Lagerelements (Lagerzapfens/Lagerbolzens/Lagerstifts) und die davon durchgriffene konische/(mehrfach) gestufte Durchgangsöffnung eine Art Gleitlager ausbildend (spielfrei) aneinander anliegen, sodass also eine Führung der Branchen zueinander in bestimmungsgemäßer Weise, d.h. insbesondere mit dem gewünschten Gangmaß, besteht. In anderen Worten ausgedrückt bewirkt insbesondere die (teilweise) konische Form des Lagerelementkopfs und die dementsprechend konische Form der Durchgangsöffnung, dass eine Kraftkomponente in Längsrichtung des Lagerelements für ein gleitfähiges Aneinanderdrücken der beiden Instrumentenbranchen und eine Kraftkomponente senkrecht zur Längsrichtung des Lagerelements erzeugt wird, wodurch Lagerspiel in Radialrichtung des Lagerelements verringert/eliminiert wird.

Die Reinigungsstellung liegt nach der Erfindung außerhalb des Arbeitsschwenkbereichs. In ihr nehmen die beiden Instrumentenbranchen eine solche relative Winkelstellung zueinander ein, in der das Instrument nach der Erfindung besonders gut und besonders einfach gereinigt sowie (gleichermaßen) sterilisiert, werden kann. Die Reinigungsstellung kann im Rahmen der Erfindung insbesondere in einem Reinigungsbereich liegen. Es handelt sich bei der Reinigungsstellung also nicht zwingend um eine konkrete (Winkel-)Stellung der beiden Branchen zueinander, sondern um eine(n) Stellung/Stellungsbereich außerhalb des Arbeitsschwenkbereichs, in dem zwischen den Branchen und vorzugsweise zwischen dem Lagerelementkopf und der davon durchgriffenen Durchgangsöffnung das erfindungsgemäß vorgesehene Spiel bzw. Spiele vorliegt / vorliegen.

Die Branchen des Instruments sind bevorzugt in einem sogenannten "aufgelegten Schluss" miteinander gekoppelt. Das heißt, dass die beiden Instrumentenbranchen bevorzugt übereinander (nicht ineinander) angeordnet sind, so dass die eine Instrumentenbranche auf der anderen Instrumentenbranche aufliegt (Bauformen "aufgelegt", "halb-aufgelegt", "eingelegt" sind aber auch möglich). In dieser bevorzugten Konstellation sind die beiden Instrumentenbranchen durch das Lagerelement miteinander gekoppelt. Das Lagerelement definiert dabei eine Schwenkachse, um die die beiden Instrumentenbranchen bei einem Verschwenken relativ zueinander drehen. Unter dem Kopplungs-/Schlussabschnitt ist derjenige Abschnitt einer Instrumentenbranche zu verstehen, der bei vollständig geschlossenem Instrument, also bei einem Öffnungswinkel von 0° zwischen den Branchen, von der anderen Instrumentenbranche im Wesentlichen vollständig verdeckt ist. Die Instrumentenbranchen sind also über dem Schlussabschnitt bevorzugt aufgelegt und verschränkt (in der Bauform "Aufgelegt" auch über die komplette Instrumentenlänge möglich).

Wenn und solange die beiden Instrumentenbranchen im Arbeitsschwenkbereich zueinander positioniert sind, liegen sie an ihren Kontaktflächen (gleitend) aneinander. Außerdem kontaktiert der Kopf des Lagerelements die Innenwandungen der Durchgangsöffnung (im Wesentlichen) spielfrei.

Sowohl im Arbeitsschwenkbereich als auch in der Reinigungsstellung ist nach der Erfindung das Lagerelement in der Durchgangsöffnung, also im Langloch, zwar drehbar oder drehpositionierbar, aber nicht translatorisch verschiebbar aufgenommen, ist also in Richtung der Längsachse des Langlochs nicht darin translatorisch relativpositionierbar.

Das Lagerelement ist derart ausgebildet und auf die beiden Instrumentenbranchen abgestimmt, dass diese innerhalb des Arbeitsschwenkbereichs in der bestimmungsgemäßen Weise und mit dem bestimmungsgemäßen Gangmaß (Drehwiderstand) aneinander gleitend anliegen und gelagert sind. Erfindungsgemäß ist das Lagerelement (Lagerstift) an der ersten Instrumentenbranche fixiert. Die Lage (axiale Lage bezogen auf die Längsachse des Lagerelements, gleichgerichtet mit der Schwenkachse) und Neigung der Konusfläche seines konischen Kopfs ist auf die Lage und Neigung der Innenkonusfläche der konischen Durchgangsöffnung der zweiten Instrumentenbranche abgestimmt. Man kann sagen, dass zwischen dem konischen Kopf des Lagerelements und der Innenkonusfläche der zweiten Instrumentenbranche in Richtung der Schwenkachse / Längsachse des Lagerelements eine (im Wesentlichen) spielfreie Passung vorliegt, deren Stärke das Gangmaß des Instruments bestimmt. Je stärker die beiden Instrumentenbranchen durch die aneinander anliegenden Konusflächen von Lagerelement und Durchgangsöffnung in der zweiten Instrumentenbranche gegeneinander gepresst oder gegeneinander verspannt werden, desto größer ist das Gangmaß. Vorzugsweise sind die beiden Instrumentenbranchen durch das Lagerelement im Arbeitsschwenkbereich (im Wesentlichen) spielfrei miteinander gekoppelt. Die konische Außenfläche des Lagerelementkopfs und die konische Innenfläche der Durchgangsöffnung der zweiten Instrumentenbranche bilden eine Art Gleitlagerung aus und gleiten bei einem Schwenken der Instrumentenbranchen zueinander aufeinander ab.

Das Lagerelement führt nur im oberen Bereich (Konus) als Gleitlager und ist in der gegenüberliegenden ersten Instrumentenbranche vorzugsweise gewindelos fixiert, wobei natürlich auch ein Gewinde zur Fixierung des Lagerelements vorstellbar ist. Durch die bevorzugt gewindelose Fixierung können die vorstehend erläuterten Nachteile des Stands der Technik besonders gut vermieden werden. Insbesondere kann das Zusammenfügen und Koppeln der beiden Instrumentenbranchen miteinander automatisch und kraft- und/oder, im Fall eines Gewindes, momentgesteuert (momentgesteuerte Schraubverbindung) durchgeführt werden, so dass die Erfindung den Vorteil ermöglicht, dass ein solches Instrument nicht länger manuell zu fügen ist. Durch beispielsweise kraftgesteuertes Einpressen eines Lagerelements z.B. in Form eines Niets bei gleichzeitigem Vernieten können jegliche Fertigungstoleranzen ausgeglichen werden. Außerdem wirkt der Nietkopf eines derartigen Niets in der Lagerstelle immer mit der gleichen Kraft (Vorspannung) und der Instrumentenschluss besitzt immer denselben Spalt bzw. ist identisch spielfrei.

Nach der Erfindung ist vorgesehen, dass die Durchgangsöffnung in der zweiten Instrumentenbranche als Langloch ausgebildet ist, dessen Längsachse sich in Längsrichtung der zweiten Instrumentenbranche erstreckt. Eine solche Durchgangsöffnung ermöglicht in der Reinigungsstellung in vorteilhafter Weise eine Verringerung der sich überlappenden Flächen beider Instrumentenbranchen auf ein Minimum und damit eine weitere Verbesserung der Reinigbarkeit. Außerdem können die schlechter zugänglichen Abschnitte der Branchen im Schlussabschnitt besonders einfach einer Sichtkontrolle unterzogen werden.

Vorteilhafte Ausführungsformen der Erfindung sind auch in den Unteransprüchen beansprucht und werden nachfolgend näher erläutert.

Eine Ausführungsform der Erfindung kann vorsehen, dass sich die Längsachse des Langlochs in Längsrichtung (L) der zweiten Instrumentenbranche erstreckt und insbesondere das Langloch bei in der Reinigungsstellung befindlichen Instrumentenbranchen in Breitenrichtung beidseitig über die erste Instrumentenbranche hinausragt.

Die Länge des Langlochs in Längsrichtung (L) kann insbesondere größer sein als die Breite (B) der ersten Instrumentenbrache. Alternativ oder zusätzlich kann die Länge des Langlochs in Längsrichtung (L) größer sein als die Länge der jeweiligen Kontaktflächen in Längsrichtung (L).

Eine Ausführungsform ist dadurch gekennzeichnet, dass bei einem Verschwenken der Instrumentenbranchen (2, 3) das Langloch (10) wenigstens 90%, vorzugsweise 95% und bevorzugter 100% der Kontaktfläche (15) der ersten Instrumentenbranche (2) überstreicht.

Nach einer Ausführungsform kann sich der Arbeitsschwenkbereich von einem Öffnungswinkel zwischen den Instrumentenbranchen von 0° bis zu einem Öffnungswinkel von zwischen ca. 50° und 40°, vorzugsweise von ca. 45° erstrecken. Durch einen derart ausgebildeten Arbeitsschwenkbereich kann unter anderem ein versehentliches Überführen des Instruments aus dem Arbeitsschwenkbereich in die Reinigungsstellung während eines Einsatzes des Instruments vermieden werden.

Es ist besonders vorteilhaft, wenn zwischen den Instrumentenbranchen in der Reinigungsstellung ein Öffnungswinkel von zwischen ca. 50° und ca. 95°, vorzugsweise von zwischen 60° und 90°, besonders bevorzugt von ca. 90° vorliegt. Dies vereinfacht ein Überführen des Instruments aus dem Arbeitsschwenkbereich in die Reinigungsstellung.

Nach einer Ausführungsform kann das Lagerelement als Lagerstift oder Lagerniet ausgebildet sein, mit einem (im Wesentlichen) zylinderförmigen Stiftabschnitt einerseits und den sich daran anschließenden aufweitenden konischen Kopf. Ein solches Lagerelement ist besonders einfach an oder in der ersten Instrumentenbranche zu fixieren. Zum Beispiel kann der Stiftabschnitt in eine Öffnung, insbesondere eine Durchgangsöffnung, der ersten Instrumentenbranche eingepresst sein. Insbesondere kann der Stiftabschnitt mit der ersten Instrumentenbranche kraftschlüssig verbunden sein, insbesondere verpresst, vernietet, verstemmt oder verklemmt sein. Alternativ oder zusätzlich kann er stoffschlüssig mit der Instrumentenbranche verbunden sein, insbesondere verklebt, verlötet oder verschweißt sein. Schließlich kann der Lagerstift auch eingeschraubt und anschließend optional verstemmt, verklebt oder verschweißt sein, um ein unbeabsichtigter Aufdrehen der Schraubverbindung zu vermeiden.

Eine Ausführungsform der Erfindung ist dadurch gekennzeichnet, dass das Langloch einen Aufnahmeabschnitt aufweist, dessen Breite gegenüber der Breite A des Langlochs aufgeweitet ist, und der andere Endabschnitt des Lagerelements in dem Aufnahmeabschnitt drehpositionierbar aufgenommen ist und einen Durchmesser aufweist, der größer als die Breite A des Langlochs ist. Der Aufnahmeabschnitt des Langlochs kann insbesondere kontinuierlich konisch oder gestuft konisch ausgebildet sein.

Die Durchgangsöffnung der zweiten Instrumentenbranche auf der dem Schlussabschnitt zugewandten Seite (auf der der ersten Instrumentenbranche zugewandten Seite) kann insbesondere einen im Wesentlichen zylinderförmigen Öffnungsabschnitt aufweisen, der gegenüber dem Lagerelement Spiel besitzt. Derart kann einfach ein Verklemmen der zweiten Instrumentenbranche mit der Konusfläche des Lagerelementkopfs verhindert werden. Zudem ist eine Durchspül-/Reinigbarkeit des zylindrischen Stiftabschnitts auch innerhalb der Durchgangsöffnung gewährleistet. Eine besondere Ausführungsform der Erfindung ist dadurch gekennzeichnet, dass der Konus-/Kegelwinkel (Spitzenwinkel) zwischen ca. 35° und ca. 55°, vorzugsweise zwischen ca. 40° und 50°, besonders bevorzugt bei ca. 45°, liegt.

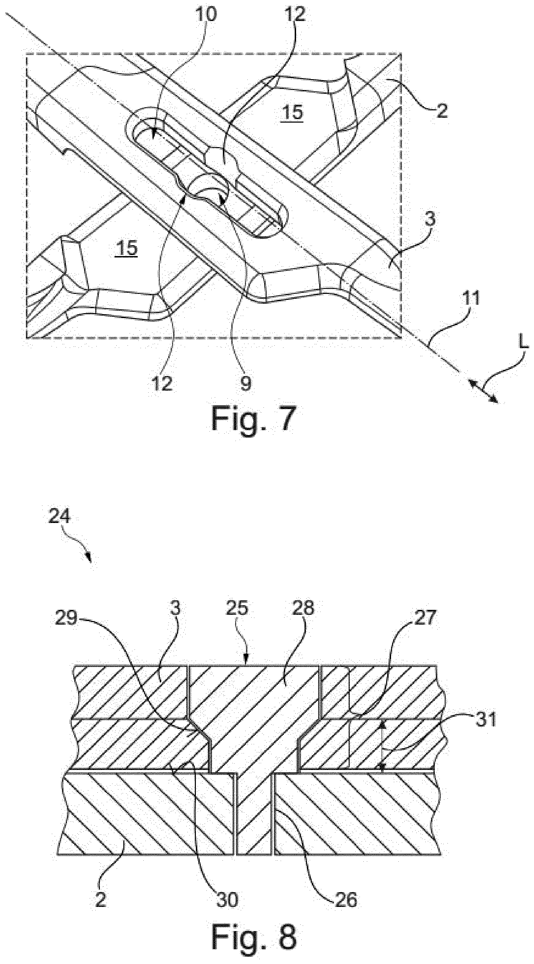

Eine weitere Ausführungsform ist eine zylindrische Senkung mit konischem Auslauf (vgl. Schraubenkopf Fig. 8). Hierbei stützt sich das Verbindungselement jedoch nicht über einen Bund an der gegenüberliegenden Instrumentenbranche ab. Es dienen nur die konischen Flächen zur Lagerung.

Eine weitere Ausführungsform ist dadurch gekennzeichnet, dass der Schlussabschnitt zumindest einer der Instrumentenbranchen eine Quernut und beiderseits der Quernut jeweils eine Kontaktfläche für den Schlussabschnitt der anderen Instrumentenbranche aufweist. Zwischen den Instrumentenbranchen liegt dann in einer Reinigungsstellung außerhalb des Arbeitsschwenkbereichs ohne gegenseitigen Kontakt über die Kontaktflächen ein Spalt oder Spiel vor. Die (aus dem Stand der Technik per se bekannte) Quernut (einseitig wechselseitig oder beidseitig) ist derart in den Kopplungs-/Schlussabschnitt eingebracht, dass sie gegenüber von an der entsprechenden Branche (Instrumententeil) (beiderseits) ausgebildeten Kontaktflächen vertieft ist und die andere Branche (Instrumententeil) in einer Reinigungs-(Winkel-)Stellung zumindest teilweise aufnehmen kann. Die Kontaktflächen beider Instrumentenbranchen sind demzufolge derart ausgebildet, dass diese im gesamten Arbeitsschwenkbereich (Arbeitsschwenkwinkelbereich) im Wesentlichen spielfrei aneinander anliegen und sich insbesondere mit dem gewünschten Gangmaß (Drehwiderstand) gegenseitig lagern/stützen.

Die Quernut ist in den Schlussabschnitt zumindest der einen jeweiligen Branche eingebracht. Man kann sagen, dass sie in die Seite oder Fläche der zumindest einen Branche eingebracht ist, die der anderen Branche zugewandt ist. Die Quernut ist (in ihrer Nutbreitenrichtung) derart ausgebildet/bemessen, dass sie die gegenüberliegende Instrumentenbranche bzw. deren Schlussabschnitt in der Reinigungsstellung (in Nutentiefenrichtung) zumindest teilweise aufnimmt. Ihre Breite entspricht daher zumindest der Breite der anderen, gegenüberliegenden Instrumentenbranche in diesem Bereich, also in deren Schlussabschnitt und ihre Nutentiefe zumindest teilweise der Dicke der gegenüberliegenden Branche. Vorzugsweise weist die Quernut eine Breite auf, die sogar geringfügig größer ist als die Breite der anderen Branche im gegenüberliegenden Schlussabschnitt, so dass deren (teilweise) Aufnahme in der Quernut auch bei nur ungefährer Positionierung der Instrumentenbranchen in der Reinigungsstellung sichergestellt ist.

Wenn und solange die beiden Instrumentenbranchen im Arbeitsschwenkbereich zueinander positioniert sind, liegt zwischen dem Quernutgrund und der gegenüberliegenden Instrumentenbranche ein Spalt vor, dessen Höhe (im Falle von in nur einer Instrumentenbranche eingebrachten Quernut) der Quernuttiefe entspricht. Im Falle von in beiden Instrumentenbranchen eingebrachten Quernuten entspricht die Höhe dieses Spalts der Summe der beiden Quernuttiefen. Die in die zumindest eine Instrumentenbranche eingebrachte Quernut beeinträchtigt die Funktion des Instruments im Arbeitsschwenkbereich nicht.

Eine Ausführungsform der Erfindung kann vorsehen, dass die Nut eine Breite (b) in Längsrichtung der jeweiligen Instrumentenbranche aufweist, die wenigstens gleich der Breite (B) der anderen Instrumentenbranche in deren Schlussabschnitt quer zu deren Längsrichtung ist, wobei die Breite (b) der Nut vorzugsweise leicht größer oder größer ist als die Breite (B) der Branche. Auf diese Weise kann eine einfache und sichere Aufnahme der anderen Instrumentenbranche in der Quernut sichergestellt werden.

Es ist besonders vorteilhaft, wenn die Nut eine Tiefe von zwischen ca. 1.0 mm bis ca. 0,1 mm, vorzugsweise von zwischen ca. 0,7 mm bis ca. 0,3 mm, besonders bevorzugt von ca. 0,5 mm aufweist. Derartige Nutabmessungen ermöglichen, dass zwischen den Instrumentenbranchen in der Reinigungsstellung ein für eine bestimmungsgemäße Reinigung ausreichendes Spiel vorliegt.

Eine andere Ausführungsform kann des Weiteren vorsehen, dass die beiden Instrumentenbranchen unmittelbar aneinander anliegen, insbesondere ohne Zwischenlage weiterer Elemente im Schlussabschnitt. Auf diese Weise kann die Teilezahl des Instruments gering gehalten werden, was eine Montage erleichtert. Außerdem ist derart sichergestellt, dass die Oberflächen der beiden Instrumentenbranchen in der Reinigungsstellung tatsächlich voneinander beabstandet sind und insbesondere nicht durch weitere Bauteile des Instruments zwischen dessen Branchen, wie zum Beispiel Distanzfedern oder Druckfedern, die für eine Beabstandung der Branchen Sorgen tragen sollen, abdeckt sind.

Man kann auch sagen, dass nach der Erfindung ohne dass zwischen den Instrumentenbranchen ein Spalt vorliegen muss eine besonders gute Möglichkeit zur Reinigung des Schlussabschnitts ermöglicht wird, indem das Lagerelement in dem Langloch unverschiebbar aufgenommen ist. Zusätzlich kann über eine Nut / Quernut (Vertiefung) auf einer oder auf beiden der innenliegenden Schlussflächen mit mind. 0,1 mm Tiefe (bisherige Erfahrung, eventuell auch weniger ausreichend je nach verdeckter Spaltfläche) im Schlussabschnitt ein Spalt erzeugt wird. Durch die Nut / Quernut ergeben sich zwei Stellungen der Instrumentenhälften zueinander: Bis ca. 45° Öffnungswinkel (Arbeitsbereich) sind die Instrumentenhälften im äußeren Bereich geführt, spielfrei und können bei Bedarf einen Gangwiderstand erzeugen. Über ca. 45° Öffnungswinkel (Reinigungsstellung) befindet sich die Nut / Quernut unter der gegenüberliegenden Instrumentenhälfte, wodurch sich Spiel im Schluss ergibt. Dadurch kann der Schlussabschnitt samt Führungsflächen gut durchspült, gereinigt und sterilisiert werden. Zusätzlich kann sich in einer Instrumentenhälfte ein Langloch befinden, das eine gute Sichtkontrolle der Reinigung im Schlussabschnitt ermöglicht. Durch das Langloch wird zudem die Reinigbarkeit / Durchspülbarkeit des Schlusses verbessert. Das Verbindungselement besitzt einen konischen Kopf, welcher in einer Kegelsenkung auf der Oberseite der Instrumentenhälfte geführt ist. Die Konuslagerung zwischen dem Lagerelement und der einen Instrumentenhälfte bewirkt eine Drehführung für eine definierte Gangeinstellung. Insgesamt handelt es sich bei der Erfindung um einen reinigungsfreundlichen Instrumentenschluss, der den bisherigen aufgelegten Schluss (Schraubschluss) sowie Durchsteckschluss ersetzen soll und eine gute Sichtkontrolle des Reinigungserfolgs im Schlussabschnitt ermöglicht. Vorzugsweise per Nietverbindung lässt sich eine kostengünstige und prozesssichere Verbindungslösung umsetzen.

Durch die Erfindung können insbesondere die folgenden Vorteile erzielt werden:
1) Bessere Reinigbarkeit / Durchspülbarkeit des Bereichs zwischen den beiden Instrumentenbranchen sowie bessere Möglichkeit einer Sichtkontrolle des Reinigungserfolgs.
2) Einfache und prozesssichere Einstellbarkeit des "Gangs" (Drehwiderstand) der Instrumentenbranchen durch definiertes Verspannen derselben.
3) Die feste Verbindung (z.B. Nietverbindung) ermöglicht eine kostengünstige Prozessautomatisierung, da das nach dem Stand der Technik manuell durchzuführende und komplexe Setzen der Schlussschraube entfällt. Nach der Erfindung kann das Lagerelement insbesondere durch folgende Verbindungstechnologien mit der einen Instrumentenhälfte fixiert sein: Vernieten, Schweißen, Pressen, Kleben, Verstemmen. Alternativ eine geschraubte Verbindung, welche nicht über den Bund gesetzt wird sondern in der benötigten Position gesichert wird (Schweißen, Kleben, Verstemmen etc.).

### Kurzbeschreibung der Figuren

Die Erfindung wird nachfolgend beispielhaft mit Hilfe von Zeichnungen näher erläutert. Es zeigt dabei:
- Fig. 1: eine Aufsicht auf einen Abschnitt eines chirurgischen Instruments nach einer ersten Ausführungsform der Erfindung mit einer Nut in einer der Branchen in einem teilweise geöffneten Zustand in dem Arbeitsschwenkbereich,
- Fig. 2: eine Aufsicht auf den Abschnitt des chirurgischen Instruments der Figur 1 in einem geöffneten Zustand in der Reinigungsstellung,
- Fig. 3: die Darstellung der Figur 1 mit markierten Kontaktflächen,
- Fig. 4: das Instrument in der Stellung der Figur 2 in einer Ansicht von unten,
- Fig. 5: eine Schnittansicht des Abschnitts der Figur 1 in dem Arbeitsschwenkbereich,
- Fig. 6: eine Schnittansicht des Abschnitts der Figur 1 in der Reinigungsstellung,
- Fig. 7: eine perspektivische Ansicht des Instruments in der Stellung der Figuren 2 und 4 ohne Lagerelement,
- Fig. 8: eine Schnittansicht einer in ein Instrument nach dem Stand der Technik eingeschraubten Lagerschraube,
- Fig. 9: eine der Figur 3 entsprechende Darstellung einer weiteren Ausführungsform des chirurgischen Instruments nach der Erfindung ohne Nut in den Branchen,
- Fig. 10: das Instrument nach der Ausführungsform der Figur 9 in einer Ansicht von unten und
- Fig. 11: eine perspektivische Ansicht des Instruments nach der Ausführungsform der Figuren 9 und 10 in der Stellung der Figur 10 ohne Lagerelement.

### Figurenbeschreibung

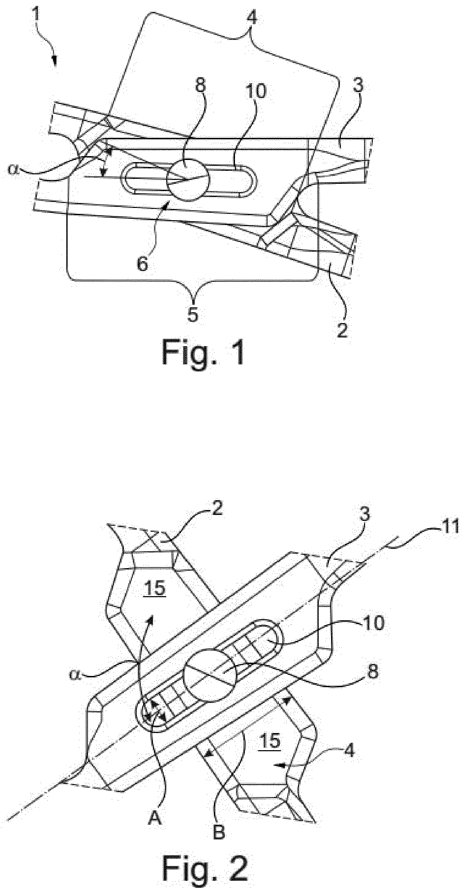

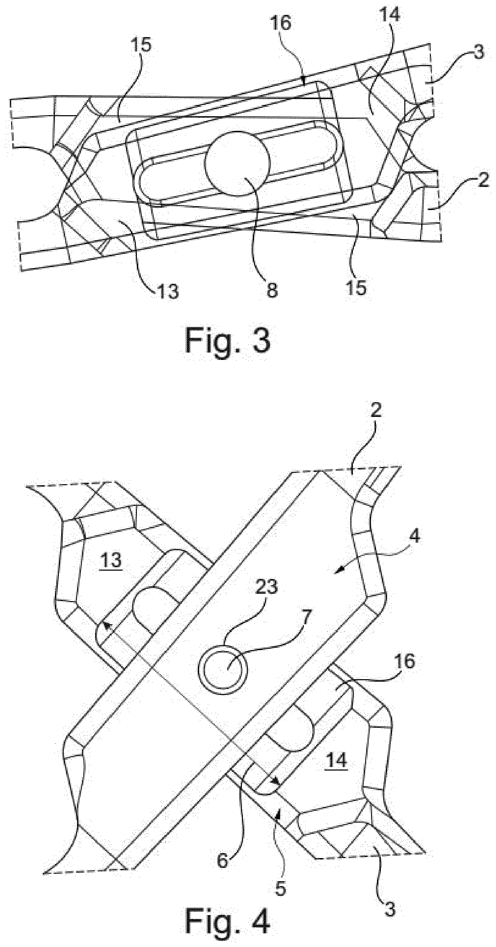

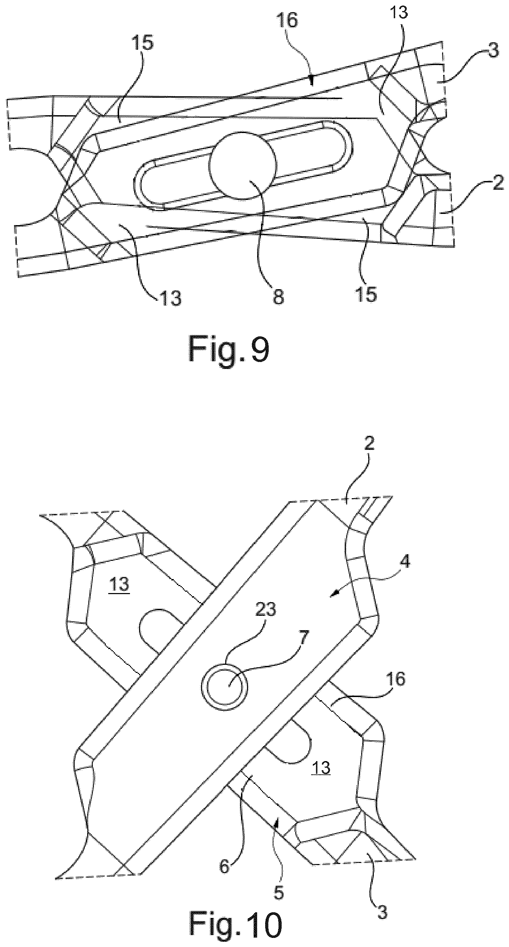

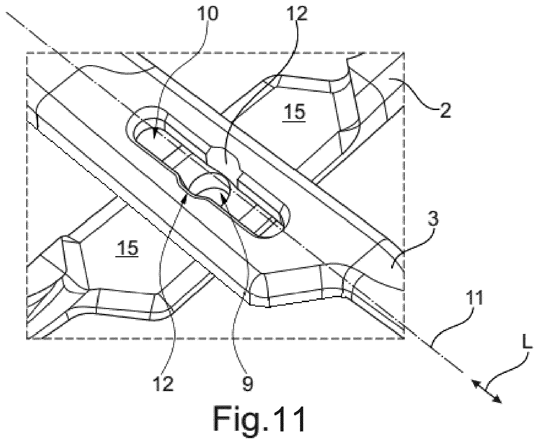

Die Zeichnungen sind lediglich schematischer Natur und dienen nur dem Verständnis der Erfindung. Die Figuren zeigen zwei Ausführungsformen: die Figuren 1 bis 7 betreffen eine erste Ausführungsform und die Figuren 1, 2, 6 und 9 bis 11 eine zweite Ausführungsform des erfindungsgemäßen Instruments. Wie insbesondere ein Vergleich der Figuren 3 mit 9 sowie 4 mit 10 ergibt, unterscheiden sich die erste und die zweite Ausführungsform darin, dass in der bei der ersten Ausführungsform in der ersten Instrumentenbranche 2 eine Quernut 16 ausgebildet ist, während eine solche Quernut in der zweiten Ausführungsform nicht vorhanden ist. Die folgende Beschreibung betrifft, soweit nicht anders angegeben, sowohl die erste als auch die zweite Ausführungsform.

Das beispielhaft in den Figuren gezeigte scheren- oder zangenartige medizintechnische Instrument 1 weist sowohl in der ersten Ausführungsform als auch in der zweiten Ausführungsform eine erste Instrumentenbranche (Instrumententeil) 2 und eine zweite Instrumentenbranche (Instrumententeil) 3 auf. Die erste Instrumentenbranche 2 verfügt über einen ersten Kopplungs-/Schlussabschnitt 4. Die zweite Instrumentenbranche 3 verfügt über einen zweiten Kopplungs-/Schlussabschnitt 5. Die beiden Instrumentenbranchen 2, 3 sind in einem sogenannten aufgelegten Schluss miteinander gekoppelt, das heißt, sie sind übereinander (nicht ineinander) angeordnet, so dass die eine, erste Instrumentenbranche 2 auf der anderen, zweiten Instrumentenbranche 3 (einseitig) aufliegt.

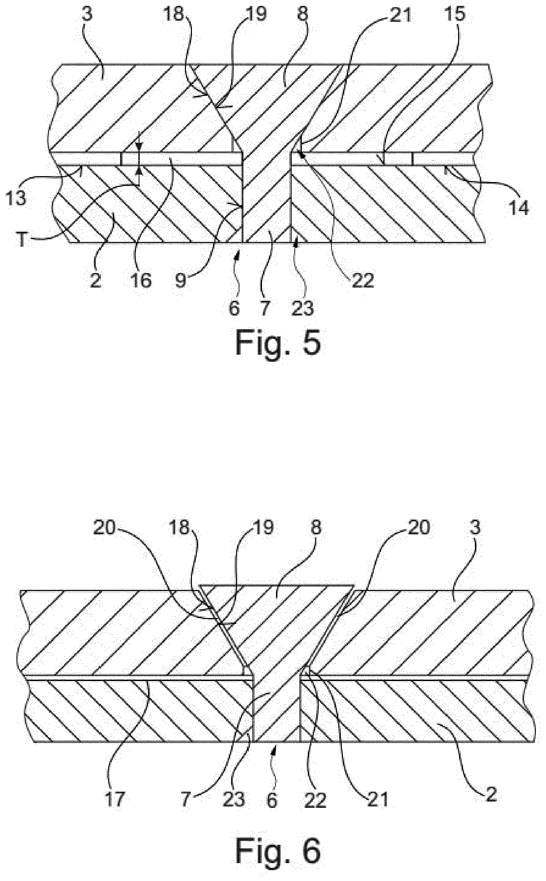

In dieser Konstellation sind die beiden Instrumentenbranchen 2, 3 durch ein Lagerelement 6 miteinander gekoppelt. Das Lagerelement 6 ist in einer Schnittansicht in den Figuren 5 und 6 gezeigt. Es als Lagerstift 6 ausgebildet und hat einen im Wesentlichen zylinderförmigen Stiftabschnitt 7 einerseits und einen axial sich daran anschließenden aufweitenden konischen Kopf 8 auf seiner dem Stiftabschnitt 7 gegenüberliegenden Axial-Seite. Er ist erfindungsgemäß gewindelos ausgebildet und mit seinem Stiftabschnitt 7 unmittelbar an der ersten Instrumentenbranche 2 fixiert. Dazu ist der Stiftabschnitt 7 bevorzugt in eine in Figur 7 erkennbare Durchgangsöffnung/Bohrung 9 der ersten Instrumentenbranche 2 eingebracht. Der Stiftabschnitt 7 kann mit der ersten Instrumentenbranche 2 kraftschlüssig verbunden, beispielsweise verpresst, vernietet, verstemmt oder verklemmt, oder stoffschlüssig verbunden, insbesondere verklebt, verlötet oder verschweißt sein. Im vorliegenden Ausführungsbeispiel ist er in die Durchgangsöffnung 9 eingepresst und endseitig auf der von der zweiten Instrumentenbranche 3 abgewandten Seite vernietet und zu einem Nietkopf 23 (zusätzlich zu dem konischen Kopf 8) ausgebildet. Zwischen der Durchgangsöffnung 9 und dem Stiftabschnitt 7 besteht eine spaltfreie Übermaßpassung.

Eine Durchgangsöffnung 10 in der zweiten Instrumentenbranche 3 ist als Langloch 10 ausgebildet. Dessen Längsverlauf 11 erstreckt sich in Längsrichtung L der zweiten Instrumentenbranche 3. Die Durchgangsöffnung 10 ist konisch ausgebildet, derart, dass sie sich in einem Abschnitt 12 mit zunehmendem Abstand von ihrer der ersten Instrumentenbranche 2 zugewandten Seite konisch aufweitet. Der konische Abschnitt 12 bildet eine der Form des Kopfs 8 der Lagerstifts 6 entsprechende Aufnahmevertiefung 12 für diesen aus. Die Aufnahmevertiefung 12 ist nicht nur konisch ausgebildet, sondern außerdem gegenüber der Breite A des Langlochs 10 in radialer Richtung aufgeweitet.

Der konische Kopf 8 des Lagerelements 6 weitet sich mit zunehmendem Abstand von der ersten Instrumentenbranche 2 bzw. mit zunehmendem Abstand vom Lagerstift 7 auf. Er durchgreift die in Figur 7 gut erkennbare konisch aufgeweitete Durchgangsöffnung 10 der zweiten Instrumentenbranche 3 und steht mit der aufgeweiteten Aufnahmevertiefung 12 in (flächiger) Anlage. Die konische Außenfläche 18 des Kopfs 8 und die konische Innenfläche 19 der Aufnahmevertiefung 12 bilden dabei bei einem Verschwenken der Branchen 2, 3 aneinander oder aufeinander abgleitende Gleitlagerflächen 18, 19. Figur 5 zeigt, dass das Langloch 10 auf seiner dem Schlussabschnitt 5 zugewandten Seite (auf der der ersten Instrumentenbranche 2 zugewandten Seite) einen im Wesentlichen zylinderförmigen Öffnungsabschnitt 21 aufweist, der gegenüber dem Lagerelement 6 und dessen Kopf 8 Spiel 22 besitzt.

Die beiden Instrumentenbranchen 2, 3 sind durch ihre Kopplung mittels des Lagerelements 6 relativ zueinander schwenkbar.

Bei der ersten Ausführungsform kontaktieren sich die Instrumentenbranchen 2, 3 in einem Arbeitsschwenkbereich über drei einander zugewandte Kontaktflächen 13, 14, 15. Im zweiten Schlussabschnitt 5 der zweiten Instrumentenbranche 3 sind eine erste Kontaktfläche 13 und eine zweite Kontaktfläche 14 ausgebildet. Im ersten Schlussabschnitt 4 der ersten Instrumentenbranche 2 ist eine dritte Kontaktfläche 15 ausgebildet. Außerdem ist im zweiten Schlussabschnitt 5 der zweiten Instrumentenbranche 3 die Quernut 16 ausgebildet, derart, dass beiderseits der Quernut 16 jeweils eine der Kontaktflächen 13, 14 angeordnet ist.

Bei der zweiten Ausführungsform kontaktieren sich die Instrumentenbranchen 2, 3 über zwei einander zugewandte Kontaktflächen 13, 15. Im zweiten Schlussabschnitt 5 der zweiten Instrumentenbranche 3 ist nur die erste Kontaktfläche 13 ausgebildet. Im ersten Schlussabschnitt 4 der ersten Instrumentenbranche 2 ist die weitere Kontaktfläche 15 ausgebildet. Außerdem ist im zweiten Schlussabschnitt 5 der zweiten Instrumentenbranche 3 anders als bei der ersten Ausführungsform keine Quernut 16 ausgebildet.

Bei der ersten Ausführungsform liegen die beiden Instrumentenbranchen 2, 3 in einem Arbeitsschwenkbereich mit ihren jeweiligen Kontaktflächen 13 und 14 bzw. 15 lagernd und führend aneinander an. Figur 3 deutet an, dass sich die Kontaktflächen 13, 14 gegenüber der dritten Kontaktfläche 15 bei einem Verschwenken der beiden Instrumentenbranchen 2, 3 relativ zueinander verschieben. Solange zwischen der ersten Kontaktfläche 13 bzw. der zweiten Kontaktfläche 14 einerseits und der dritten Kontaktfläche 15 andererseits Kontakt besteht, diese also aneinander anliegen, sind die beiden Instrumente zueinander geführt und befinden sich im Arbeitsschwenkbereich. Lösen sich die Kontaktflächen 13, 14, 15 voneinander, befindet sich das Instrument 1 nicht mehr im Arbeitsschwenkbereich. Das sich im Arbeitsschwenkbereich befindliche Instrument 1 nach der ersten Ausführungsform ist in den Figuren 1, 3 und 5 gezeigt.

Außerhalb des Arbeitsschwenkbereichs kann das Instrument 1 nach der ersten Ausführungsform in eine Reinigungsstellung gebracht werden. In dieser liegt zwischen den Instrumentenbranchen 2, 3 und insbesondere zwischen deren Schlussabschnitten 4, 5 ohne gegenseitigen Kontakt über die Kontaktflächen 13, 14, 15 ein Spalt 17 oder ein Spiel 17 vor. Außerdem liegt zwischen den Gleitlagerflächen 18, 19, also zwischen dem Kopf 8 des Lagerelements 6 und dem Langloch 10, insbesondere der Aufnahmevertiefung 12 ein Spalt 20 oder ein Spiel 20 vor. Das in seiner Reinigungsstellung befindliche Instrument 1 ist in den Figuren 2, 4, 6 und 7 gezeigt.

Die Quernut 16 der ersten Ausführungsform besitzt eine Breite (b) in Längsrichtung L der zweiten Instrumentenbranche 3, die wenigstens gleich der Breite (B) der ersten Instrumentenbranche 2 in deren Schlussabschnitt 4 quer zu deren Längsrichtung ist. Im vorliegenden Beispiel ist die Breite (b) der Quernut 16 größer ist als die Breite (B) der Branche 2, siehe insbesondere in Figur 4. Die Quernut 16 weist eine Tiefe T auf, siehe in Figur 5. Die Tiefe T der Quernut 16 ist derart bemessen, dass zwischen den beiden Schlussabschnitten 4, 5 der in der Reinigungsstellung befindlichen Instrumentenbranchen 2, 3 (und damit auch zwischen den Kontaktflächen 13, 14, 15) der Spalt 17 ausgebildet wird und vorliegt. Außerdem sind die Tiefe T der Quernut 16 und die Aufnahmevertiefung 12 im Langloch 10 derart aufeinander abgestimmt, dass sich der konische Kopf 8 aus der Aufnahmevertiefung 12 lösen kann.

Figur 8 zeigt ein bekanntes Instrument 24, bei denen eine Schlussschraube 25 auf Anschlag (Bund) in eine der Instrumentenbranchen 2 eingeschraubt ist und zur Sicherung leicht überdreht ist, wodurch es zu einem Kaltverschweißen der Gewindegänge 26 kommt. Die Schlussschraube 25 durchgreift eine Stufenbohrung 27 in der anderen Instrumentenbranche 3 und weist einen Schraubenkopf 28 auf, der an der anderen Instrumentenbranche 3 zur Anlage kommt und diese in Richtung der einen Instrumentenbranche 2 drängt / verspannt. Der Gang des Instruments 24 ergibt sich (resultiert aus Verspannung der Instrumentenbranchen 2, 3 gegeneinander) aus dem Abstand der Anlagefläche 29 des Schraubenkopfs 28 zur Kontaktfläche 30 der einen Instrumentenbranche 2 sowie den Toleranzen 31 der Stufenbohrung 27 der anderen Instrumentenbranche 3. Dadurch ist die Verschraubung überbestimmt.

Bei der zweiten Ausführungsform liegen die beiden Instrumentenbranchen 2, 3 sowohl in einem Arbeitsschwenkbereich wie auch in der Reinigungsstellung mit ihren jeweiligen Kontaktflächen 13 bzw. 15 lagernd und führend aneinander an.

Die Breite des Kopfs 8 ist außerdem größer als die Breite A des Langlochs 10, so dass das Lagerelement 6 zwar in der Aufnahmevertiefung 12 drehbar ist, allerdings nicht translatorisch in dem Langloch 10 in dessen Längsrichtung positioniert werden kann, also aus der Aufnahmevertiefung 12 herausgelangen kann. Derart sind bei der zweiten Ausführungsform die beiden Instrumentenbranchen 2, 3 rein (ausschließlich) drehbar miteinander gekoppelt. Eine Möglichkeit zur Reinigung der Kontaktflächen 13, 14, 15 besteht schon darin, dass das Langloch 10 diese beim Verschwenken der Instrumentenbranchen 2, 3 quasi überstreicht und diese durch das Langloch 10 zur Reinigung zugänglich sind.

### Bezugszeichenliste

- 1: Instrument
- 2: erste Instrumentenbranche
- 3: zweite Instrumentenbranche
- 4: erster Schlussabschnitt
- 5: zweiter Schlussabschnitt
- 6: Lagerelement, Lagerstift
- 7: Stiftabschnitt
- 8: konischer Kopf
- 9: Durchgangsöffnung
- 10: Durchgangsöffnung, Langloch
- 11: Längsachse
- 12: konischer Abschnitt, Aufnahmevertiefung
- 13: erste Kontaktfläche
- 14: zweite Kontaktfläche
- 15: dritte Kontaktfläche
- 16: Quernut
- 17: Spalt, Spiel
- 18: Außenfläche, Gleitlagerfläche
- 19: Innenfläche, Gleitlagerfläche
- 20: Spalt, Spiel
- 21: zylinderförmiger Abschnitt
- 22: Spiel
- 23: Nietkopf
- 24: Instrument (Stand der Technik)
- 25: Schlussschraube
- 26: Gewindegänge
- 27: Stufenbohrung
- 28: Schraubenkopf
- 29: Anlagefläche
- 30: Kontaktfläche
- 31: Toleranzen
- A: Breite Langloch 10
- B: Breite Schlussabschnitt 4
- b: Breite Quernut 16
- L: Längsrichtung

## Patentansprüche

1. Scheren- oder zangenartiges medizintechnisches Instrument (1) mit einer ersten Instrumentenbranche (2), einer zweiten Instrumentenbranche (3) und einem die Instrumentenbranchen (2, 3) in einem aufgelegten Schluss koppelnden Lagerelement (6),
wobei die beiden Instrumentenbranchen (2, 3) relativ zueinander schwenkbar sind und sich in einem Arbeitsschwenkbereich über einander zugewandte Kontaktflächen (13, 14, 15) in einem Schlussabschnitt (4, 5) gegenseitig kontaktieren,
so dass die Instrumentenbranchen (2, 3) in dem Arbeitsschwenkbereich aneinander mit ihren jeweiligen Kontaktflächen (13, 14, 15) lagernd und führend anliegen,
und die Instrumentenbranchen (2, 3) in eine Reinigungsstellung außerhalb des Arbeitsschwenkbereichs positionierbar sind,
wobei das Lagerelement (6) vorzugsweise gewindelos ist und an einem Endabschnitt unmittelbar an der ersten Instrumentenbranche (2) fest fixiert ist und am anderen Endabschnitt einen sich mit zunehmendem Abstand von der ersten Instrumentenbranche (2) kontinuierlich konisch oder gestuft konisch aufweitenden Kopf (8) aufweist, der eine kontinuierlich oder gestuft konische Durchgangsöffnung (10) in der zweiten Instrumentenbranche (3) durchgreift, wobei die Durchgangsöffnung (10) in Form eines Langlochs (10) ausgebildet ist, **dadurch gekennzeichnet, dass** das Lagerelement (6) in dem Langloch (10) drehpositionierbar und translatorisch fest aufgenommen ist, insbesondere in Längsrichtung (L) des Langlochs (10) positionsfest aufgenommen ist.

2. Instrument nach Anspruch 1, **dadurch gekennzeichnet dass** das Langloch (10) vorzugsweise in seinem Längsmittenabschnitt eine weiter vorzugsweise kreisförmige Ausbauchung oder Einkerbung (12) an einer Längsflanke des Langlochs oder in gegenüberliegender Weise an beiden Längsflanken des Langlochs hat, mit der oder denen das Lagerelement (6) in einen eine Verschiebung längs des Langlochs (10) blockierenden Eingriff steht.

3. Instrument (1) nach Anspruch 1 oder 2, **dadurch gekennzeichnet, dass** sich die Längsachse des Langlochs (10) in Längsrichtung (L) der zweiten Instrumentenbranche (3) erstreckt und insbesondere das Langloch (10) bei in der Reinigungsstellung befindlichen Instrumentenbranchen (2, 3), in welcher die Instrumentenbranchen (2, 3) im Wesentlichen rechtwinklig zueinander stehen, in Breitenrichtung beidseitig über die erste Instrumentenbranche (2) hinausragt.

4. Instrument (1) nach Anspruch 1, 2 oder 3, **dadurch gekennzeichnet, dass** die Länge des Langlochs (10) in Längsrichtung (L) größer ist als die Breite (B) der ersten Instrumentenbrache (2) und/oder die Länge des Langlochs (10) in Längsrichtung (L) größer als die Länge der jeweiligen Kontaktflächen (13, 14, 15) in Längsrichtung (L).

5. Instrument nach einem der vorstehenden Ansprüche, **dadurch gekennzeichnet, dass** bei einem Verschwenken der Instrumentenbranchen (2, 3) das Langloch (10) wenigstens 90%, vorzugsweise 95% und bevorzugter 100% der Kontaktfläche (15) der ersten Instrumentenbranche (2) überstreicht.

6. Instrument nach einem der vorstehenden Ansprüche, **dadurch gekennzeichnet, dass** sich der Arbeitsschwenkbereich von einem Öffnungswinkel α zwischen den Instrumentenbranchen (2, 3) von 0° bis zu einem Öffnungswinkel von zwischen ca. 50° und 40°, vorzugsweise von ca. 45° erstreckt und/oder zwischen den Instrumentenbranchen (2, 3) in der Reinigungsstellung ein Öffnungswinkel α von zwischen ca. 50° und ca. 95°, vorzugsweise von zwischen 60° und 90°, besonders bevorzugt von ca. 90° vorliegt.

7. Instrument (1) nach einem der vorstehenden Ansprüche, **dadurch gekennzeichnet, dass** das Lagerelement (6) als Lagerstift (6) ausgebildet ist, mit einem im Wesentlichen zylinderförmigen Stiftabschnitt (7) einerseits und dem sich daran anschließenden Kopf (8), wobei vorzugsweise der Stiftabschnitt (7) in eine Öffnung (9), insbesondere eine Durchgangsöffnung (9), der ersten Instrumentenbranche (2) eingepresst ist.

8. Instrument (1) nach Anspruch 6, **dadurch gekennzeichnet, dass** der Stiftabschnitt (7) mit der ersten Instrumentenbranche (2) kraftschlüssig verbunden ist, insbesondere verpresst, vernietet, verstemmt oder verklemmt ist, oder stoffschlüssig verbunden ist, insbesondere verklebt, verlötet oder verschweißt ist, oder insbesondere der Stiftabschnitt (7) mit der ersten Instrumentenbranche (2) per Schraubverbindung verbunden ist, welche vorzugsweise drehmomentgesteuert angezogen ist.

9. Instrument (1) nach einem der vorstehenden Ansprüche 2 bis 8, **dadurch gekennzeichnet, dass** die Ausbauchungen oder Einkerbungen (12) gemeinsam einen bohrungsförmigen Aufnahmeabschnitt (12) bilden, dessen Breite gegenüber der Breite A des Langlochs (10) aufgeweitet ist, und der andere Endabschnitt des Lagerelements (6) in dem Aufnahmeabschnitt (12) drehpositionierbar aufgenommen ist und einen Durchmesser aufweist, der größer als die Breite A des Langlochs (10) ist.

10. Instrument (1) nach Anspruch 9, **dadurch gekennzeichnet, dass** der Aufnahmeabschnitt (12) des Langlochs (10) kontinuierlich konisch oder gestuft konisch ausgebildet ist.

11. Instrument (1) nach einem der vorstehenden Ansprüche, **dadurch gekennzeichnet, dass** die Durchgangsöffnung (10) der zweiten Instrumentenbranche (3) auf der dem Schlussabschnitt (5) zugewandten Seite einen im Wesentlichen zylinderförmigen Öffnungsabschnitt (21) aufweist, der gegenüber dem Lagerelement (6) Spiel (22) besitzt.

12. Instrument (1) nach einem der vorstehenden Ansprüche, **dadurch gekennzeichnet, dass** der Schlussabschnitt (5) zumindest einer der Instrumentenbranchen (3) eine Quernut (16) und beiderseits der Quernut (16) jeweils eine Kontaktfläche (13, 14) für den Schlussabschnitt (4) der anderen Instrumentenbranche (2) aufweist, wobei zwischen den Instrumentenbranchen (2, 3) in einer Reinigungsstellung außerhalb des Arbeitsschwenkbereichs ohne gegenseitigen Kontakt über die Kontaktflächen (13, 14, 15) ein Spalt (17) oder Spiel (17) vorliegt.

13. Instrument (1) nach Anspruch 12, **dadurch gekennzeichnet, dass** die Quernut (16) eine Breite (b) in Längsrichtung (L) der jeweiligen Instrumentenbranche (3) aufweist, die wenigstens gleich der Breite (B) der anderen Instrumentenbranche (2) in deren Schlussabschnitt (4) quer zu deren Längsrichtung (L) ist, wobei die Breite (b) der Quernut (16) vorzugsweise leicht größer oder größer ist als die Breite (B) der Branche (3) und/oder die Quernut (16) eine Tiefe (T) von zwischen ca. 1.0 mm bis ca. 0,1 mm, vorzugsweise von zwischen ca. 0,7 mm bis ca. 0,3 mm, besonders bevorzugt von ca. 0,5 mm aufweist.

## Claims

1. A scissor-like or forceps-like medical-technical instrument (1) with a first instrument branch (2), a second instrument branch (3), and a bearing element (6) coupling the instrument branches (2, 3) in a superimposed closure,
wherein the two instrument branches (2, 3) are pivotable relative to each other and contact each other in a closure portion (4, 5) in an operation pivot region via mutually facing contact surfaces (13, 14, 15),
so that the instrument branches (2, 3) in the operation pivot region contact each other with their respective contact surfaces (13, 14, 15) in a bearing and guiding manner, and the instrument branches (2, 3) are positionable in a cleaning position outside the operation pivot region,
wherein the bearing element (6) is preferably threadless and is securely fixed at an end portion directly on the first instrument branch (2) and has at the other end portion a head (8) widening conically in a continuous or stepped manner with increasing distance from the first instrument branch (2), said head (8) penetrating a continuously conical or stepped conical passage opening (10) in the second instrument branch (3), wherein the passage opening (10) is designed in the form of an elongated hole (10), **characterized in that** the bearing element (6) is rotatably positionable in the elongated hole (10) and translationally fixedly received, in particular fixedly received in position in the longitudinal direction (L) of the elongated hole (10).

2. Instrument according to claim 1, **characterized in that** the elongated hole (10), preferably in its longitudinal central section, has further preferably a circular bulge or notch (12) on a longitudinal flank of the elongated hole or in an opposite manner on both longitudinal flanks of the elongated hole, with which the bearing element (6) is in engagement blocking a displacement along the elongated hole (10).

3. Instrument (1) according to claim 1 or 2, **characterized in that** the longitudinal axis of the elongated hole (10) extends in the longitudinal direction (L) of the second instrument branch (3) and in particular the elongated hole (10) projects in the width direction on both sides beyond the first instrument branch (2) when the instrument branches (2, 3) are in the cleaning position, in which the instrument branches (2, 3) are substantially at right angles to each other.

4. Instrument (1) according to claim 1, 2 or 3, **characterized in that** the length of the elongated hole (10) in the longitudinal direction (L) is greater than the width (B) of the first instrument branch (2) and/or the length of the elongated hole (10) in the longitudinal direction (L) is greater than the length of the respective contact surfaces (13, 14, 15) in the longitudinal direction (L).

5. Instrument according to one of the preceding claims, **characterized in that,** when pivoting the instrument branches (2, 3), the elongated hole (10) sweeps at least 90%, preferably 95%, and more preferably 100% of the contact surface (15) of the first instrument branch (2).

6. Instrument according to one of the preceding claims, **characterized in that** the operation pivot region extends from an opening angle α between the instrument branches (2, 3) from 0° to an opening angle of between approx. 50° and 40°, preferably of approx. 45°, and/or between the instrument branches (2, 3) in the cleaning position there is an opening angle α of between approx. 50° and approx. 95°, preferably of between 60° and 90°, particularly preferably of approx. 90°.

7. Instrument (1) according to one of the preceding claims, **characterized in that** the bearing element (6) is designed as a bearing pin (6), having an essentially cylindrical pin portion (7) and the adjacent head (8), wherein preferably the pin portion (7) is pressed into an opening (9), in particular a passage opening (9), of the first instrument branch (2).

8. Instrument (1) according to claim 6, **characterized in that** the pin portion (7) is force-lockingly connected to the first instrument branch (2), in particular pressed, riveted, plastically deformed or clamped, or is integrally connected, in particular glued, soldered or welded, or in particular the pin portion (7) is connected to the first instrument branch (2) by screw connection, which is preferably tightened in a torque-controlled manner.

9. Instrument (1) according to one of the preceding claims 2 to 8, **characterized in that** the bulges or notches (12) together form a drill-shaped receiving portion (12), the width of which is widened in relation to the width A of the elongated hole (10), and the other end portion of the bearing element (6) is received in the receiving portion (12) in a rotatably positionable manner and has a diameter which is greater than the width A of the elongated hole (10).

10. Instrument (1) according to claim 9, **characterized in that** the receiving portion (12) of the elongated hole (10) is continuously conical or conically stepped.

11. Instrument (1) according to one of the preceding claims, **characterized in that** the passage opening (10) of the second instrument branch (3) has, on the side facing the closure portion (5), an essentially cylindrical opening portion (21) which has a clearance (22) with respect to the bearing element (6).

12. Instrument (1) according to one of the preceding claims, **characterized in that** the closure portion (5) of at least one of the instrument branches (3) has a transverse groove (16) and has on both sides of the transverse groove (16) a respective contact surface (13, 14) for the closure portion (4) of the other instrument branch (2), wherein a gap (17) or a clearance (17) is present between the instrument branches (2, 3) in a cleaning position outside the operation pivot region without contacting each other via the contact surfaces (13, 14, 15).

13. Instrument (1) according to claim 12, **characterized in that** the transverse groove (16) has a width (b) in the longitudinal direction (L) of the respective instrument branch (3) which is at least equal to the width (B) of the other instrument branch (2) in its closure portion (4) transversely to its longitudinal direction (L), wherein the width (b) of the transverse groove (16) is preferably slightly greater or greater than the width (B) of the branch (3) and/or the transverse groove (16) has a depth (T) of between approx. 1.0 mm to approx. 0.1 mm, preferably from between approx. 0.7 mm to approx. 0.3 mm, particularly preferably of approx. 0.5 mm.

## Revendications

1. Instrument (1) à usage médical de type ciseaux ou pinces comportant une première branche d'instrument (2), une seconde branche d'instrument (3) et un élément de support (6) couplant les branches d'instrument (2, 3) dans une entablure posée,
dans lequel les deux branches d'instrument (2, 3) peuvent pivoter l'une par rapport à l'autre et entrent en contact mutuellement dans une zone de pivotement de travail par l'intermédiaire de surfaces de contact (13, 14, 15) tournées les unes vers les autres dans une section d'entablure (4, 5),
de sorte que les branches d'instrument (2, 3) s'appuient l'une contre l'autre de manière supportée et guidée avec leurs surfaces de contact (13, 14, 15) respectives dans la zone de pivotement de travail,
et les branches d'instrument (2, 3) peuvent être positionnées dans une position de nettoyage en dehors de la zone de pivotement de travail,
dans lequel l'élément de support (6) est de préférence sans filetage et est fixé de manière solidaire au niveau d'une section d'extrémité directement sur la première branche d'instrument (2) et présente, au niveau de l'autre section d'extrémité, une tête (8) s'élargissant de manière conique en continu ou de manière conique étagée à une distance croissante par rapport à la première branche d'instrument (2), laquelle tête traverse une ouverture de passage (10) conique en continu ou étagée dans la seconde branche d'instrument (3), l'ouverture de passage (10) étant réalisée sous la forme d'un trou oblong (10),
**caractérisé en ce que** l'élément de support (6) est reçu dans le trou oblong (10) de manière à pouvoir être positionné par rotation et de manière solidaire en translation, en particulier est reçu de manière solidaire en position dans la direction longitudinale (L) du trou oblong (10).

2. Instrument selon la revendication 1, **caractérisé en ce que** le trou oblong (10) possède, de préférence dans sa section médiane longitudinale, un renflement ou une encoche (12), de préférence en outre circulaire, sur un flanc longitudinal du trou oblong ou de manière opposée sur les deux flancs longitudinaux du trou oblong, avec lequel ou lesquels l'élément de support (6) est en prise bloquant un déplacement le long du trou oblong (10).

3. Instrument (1) selon la revendication 1 ou 2, **caractérisé en ce que** l'axe longitudinal du trou oblong (10) s'étend dans la direction longitudinale (L) de la seconde branche d'instrument (3) et en particulier le trou oblong (10), lorsque les branches d'instrument (2, 3) se trouvent dans la position de nettoyage, dans laquelle les branches d'instrument (2, 3) sont sensiblement perpendiculaires l'une à l'autre, fait saillie des deux côtés depuis la première branche d'instrument (2) dans le sens de la largeur.

4. Instrument (1) selon la revendication 1, 2 ou 3, **caractérisé en ce que** la longueur du trou oblong (10) dans la direction longitudinale (L) est supérieure à la largeur (B) de la première branche d'instrument (2) et/ou la longueur du trou oblong (10) dans la direction longitudinale (L) est supérieure à la longueur des surfaces de contact (13, 14, 15) respectives dans la direction longitudinale (L).

5. Instrument selon l'une des revendications précédentes,
**caractérisé en ce que,** lors d'un pivotement des branches d'instrument (2, 3), le trou oblong (10) couvre au moins 90 %, de préférence 95 % et de manière particulièrement préférée 100 % de la surface de contact (15) de la première branche d'instrument (2).

6. Instrument selon l'une des revendications précédentes,
**caractérisé en ce que** la zone de pivotement de travail s'étend d'un angle d'ouverture α entre les branches d'instrument (2, 3) de 0° jusqu'à un angle d'ouverture compris entre environ 50° et 40°, de préférence d'environ 45°, et/ou un angle d'ouverture α compris entre environ 50° et environ 95°, de préférence compris entre 60°et 90°, plus préférablement d'environ 90°, est présent entre les branches d'instrument (2, 3) dans la position de nettoyage.

7. Instrument (1) selon l'une des revendications précédentes,
**caractérisé en ce que** l'élément de support (6) est réalisé en tant que broche de support (6) comportant une section de broche (7) sensiblement cylindrique d'un côté et la tête (8) se raccordant à celle-ci, de préférence la section de broche (7) étant enfoncée dans une ouverture (9), en particulier une ouverture traversante (9), de la première branche d'instrument (2).

8. Instrument (1) selon la revendication 6, **caractérisé en ce que** la section de broche (7) est reliée à force à la première branche d'instrument (2), en particulier est comprimée, rivetée, matée ou serrée, ou est reliée par liaison de matière, en particulier est collée, brasée ou soudée, ou en particulier la section de broche (7) est reliée à la première branche d'instrument (2) par une liaison vissée, laquelle est de préférence serrée de manière commandée par couple.

9. Instrument (1) selon l'une des revendications précédentes 2 à 8, **caractérisé en ce que** les renflements ou encoches (12) forment ensemble une section de réception (12) en forme de trou, dont la largeur est élargie par rapport à la largeur A du trou oblong (10), et l'autre section d'extrémité de l'élément de support (6) est reçue de manière à pouvoir être positionnée par rotation dans la section de réception (12) et présente un diamètre supérieur à la largeur A du trou oblong (10).

10. Instrument (1) selon la revendication 9, **caractérisé en ce que** la section de réception (12) du trou oblong (10) est réalisée de manière conique en continu ou de manière conique étagée.

11. Instrument (1) selon l'une des revendications précédentes,
**caractérisé en ce que** l'ouverture traversante (10) de la seconde branche d'instrument (3) présente, sur le côté tourné vers la section d'entablure (5), une section d'ouverture (21) sensiblement cylindrique, laquelle possède un jeu (22) par rapport à l'élément de support (6).

12. Instrument (1) selon l'une des revendications précédentes,
**caractérisé en ce que** la section d'entablure (5) d'au moins l'une des branches d'instrument (3) présente une rainure transversale (16) et, des deux côtés de la rainure transversale (16), respectivement une surface de contact (13, 14) pour la section d'entablure (4) de l'autre branche d'instrument (2), une fente (17) ou un jeu (17) étant présent entre les branches d'instrument (2, 3) dans une position de nettoyage en dehors de la zone de pivotement de travail, sans contact mutuel par l'intermédiaire des surfaces de contact (13, 14, 15).

13. Instrument (1) selon la revendication 12, **caractérisé en ce que** la rainure transversale (16) présente une largeur (b) dans la direction longitudinale (L) de la branche d'instrument (3) respective au moins égale à la largeur (B) de l'autre branche d'instrument (2) dans sa section d'entablure (4) transversalement à sa direction longitudinale (L), la largeur (b) de la rainure transversale (16) étant de préférence légèrement supérieure ou supérieure à la largeur (B) de la branche (3) et/ou la rainure transversale (16) présentant une profondeur (T) comprise entre environ 1,0 mm et environ 0,1 mm, de préférence comprise entre environ 0,7 mm et environ 0,3 mm, plus préférablement d'environ 0,5 mm.
